Europäisches Patentamt

European Patent Office

Office européen des brevets

(11) Publication number: **0 280 572**
A1

# EUROPEAN PATENT APPLICATION

(21) Application number: 88301687.5

(22) Date of filing: 26.02.88

(51) Int. Cl.⁴: **A 61 F 2/08**
A 61 B 19/00, G 01 L 5/08,
G 01 L 7/16

(30) Priority: 27.02.87 US 19578

(43) Date of publication of application:
31.08.88 Bulletin 88/35

(84) Designated Contracting States: **CH DE FR GB LI**

(71) Applicant: **SYNTHES AG**
Grabenstrasse 15
CH-7002 Chur (CH)

(72) Inventor: **Ziegler, Lloyd Lester**
1690 Russell Road
Paoli PA. 193101-1222 (US)

(74) Representative: **Newby, John Ross**
J.Y. & G.W. Johnson Furnival House 14/18 High Holborn
London WC1V 6DE (GB)

(54) **Tension isometer.**

(57) A tension isometer (1) for surgical purposes comprises a gas pressure chamber (18) with a piston (19) to which a suture (45) may be clamped.

FIG.1

EP 0 280 572 A1

Bundesdruckerei Berlin

## Description

### TENSION ISOMETER

This invention relates to a tension isometer for surgical use and in particular to a tension isometer for use in connection with the replacement or reconstruction of cruciate ligaments of the knee.

In the replacement of the anterior cruciate ligament (ACL) of the knee,it has been recognized that best results, in terms of stability of the treated knee, is obtained if the replacement suture or graft is isometrically placed, i.e., is attached to the femur and the tibia at points such that the tension on the suture is substantially constant whether the knee is flexed or not flexed.

To determine isometric points of attachment current practice calls for narrow holes to be drilled through the femur and the tibia as by a Kirschner wire. A suture is then secured to the femur and passed through the holes left by the Kirschner wires and secured to a so-called "tension isometer". This term, as presently used, refers to a strain gauge which is deflected from its initial position by an increase or decrease in the tension of the filament to which it is attached. The points of attachment of the suture on the femur and tibia which result in zero or minimal deflection are isometric points.

Current tension isometers are spring loaded. Since it is desirable to be able to adjust the zero deflection tension for different patients, in some isometers means are provided for varying the spring tension. However, it has been found that such devices are not entirely satisfactory, since the force being exerted by the spring is never very accurately known. Furthermore, the tension on the filament will not remain constant due to the varying forces developed by the spring as it is deflected from its initial position by either a greater or lesser amount of compression.

In accordance with the present invention there is provided a tension isometer which can be set for virtually any desired tension and maintained precisely at that tension. In accordance with the invention this result is obtained by means of a device of the class described comprising a gas pressure chamber, a piston slidably mounted within said chamber, means for securing a filament to said piston and means for supplying a gas under pressure to said chamber. Since the tension on the filament is always a direct and constant function of the pressure of the gas, it is capable of precise adjustment.

The invention will be further described with reference to the accompanying drawings in which:

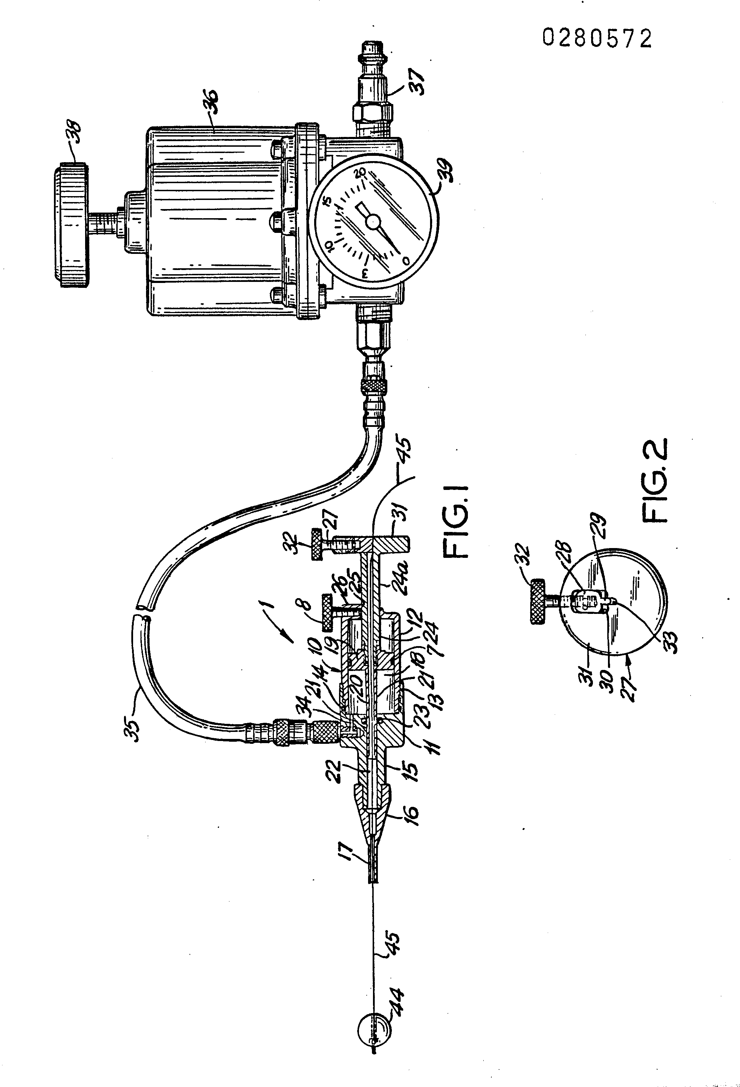

Fig. 1 is a side elevational view, partly in vertical section of an isometer according to the invention with a gas regulator for supplying constant pressure gas.

Fig. 2 is an end view of the piston and filament clamp used in the isometer of Fig. 1.

Fig. 3 is a schematic view showing the manner of using the device in the replacement of an anterior cruciate ligament.

Referring to the drawings, an isometer 1 according to the invention comprises a casing 10, normally cylindrical in shape. For convenience of manufacture and maintenance the casing 10 may be made in several sections. In the embodiment shown in the drawing there is a forward section 11 and a rear section 12 joined by a threaded connection 13 and sealed with an O-ring 14.

The forward section 11 has a nose 15, to which is fitted a detachable conical cap 16 which tapers down to a narrow tubular section 17, having a diameter sufficiently small to enable it to be fitted into the hole left by a Kirschner wire.

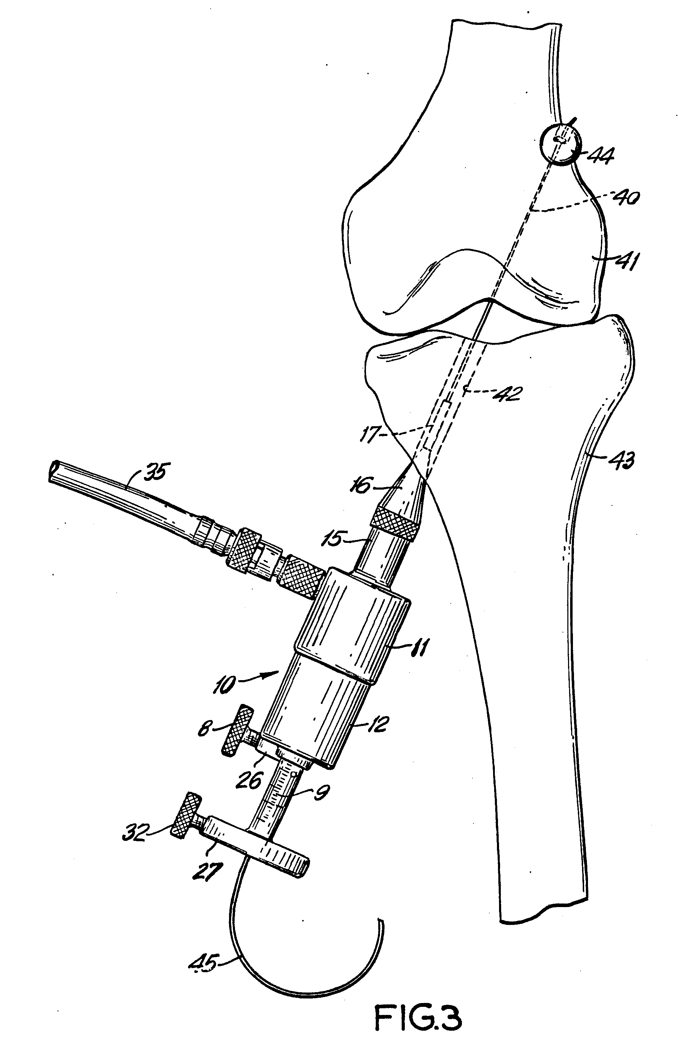

The interior of casing 10 is hollow, providing a gas chamber 18 preferably cylindrical in shape. In this chamber 18 is positioned a piston 19. As shown, the piston may be formed in two sections. The forward section 20 has a cannulated or hollow tubular forward extension 21 which slides in a passageway 22 formed in the forward section of the casing, and extending with reduced diameter through the tubular section 17. An O-ring 23 provides a gas seal for the extension 21 in the passageway 22. The rear section 24 of the piston 19 is threaded into the forward section. It comprises a hollow tubular rear extension 24a which slides through an aperture 25 in the rear wall 26 of the rear section 12 of the casing 10. As shown in the drawing there is a continuous passage extending from the tubular section 17 of the cap 16, through the nose 15 of the casing, the cannulated forward extension 21 of the piston and the cannulated rear section 24 of the piston. This passage will accommodate a filamentary element, e.g., a suture, and a screw clamp 27 is provided at the end of the rear section 24 of the piston 19 to secure such a filament to the piston. As shown in Fig. 2, the clamp comprises a block 28 having a finger 29. The block slides in an elongated socket 30 formed in the rear wall 31 of the rear section of the piston. The block 28 is attached to a screw 32 which is threaded into the rear wall 31. The socket 30 has a well 33 and the finger 29 is positioned to lock the filament in the well 33 as the screw 32 moves the block downwardly in socket 30.

The forward section 11 of the casing 10 is provided with a gas inlet 34 of conventional design to which is attached a tube 35 which in turn leads to a gas pressure regulator 36. The regulator, of conventional design, may be attached to a source of gas under pressure, e.g., a nitrogen tank (not shown), through a coupling 37. Regulator 36 is provided with a knob 38 for adjusting the pressure of the gas delivered to the isometer. A pressure dial 39 is connected to the regulator and is preferably calibrated to read pounds of force delivered to the piston and hence to a filament attached to the piston.

As shown in Fig. 3, a scale 9 may be inscribed on the rod-like rear section 24a of the piston 19. A screw clamp 8 may be positioned in the rear wall 26 of the case to lock the piston in any desired position. An O-ring 7 may be used to seal the piston in the chamber 18.

Use of the device is shown in Fig. 3. Referring to that Figure, to insert a replacement ligament a hole 40 is drilled through the femur 41, as with a Kirschner wire (not shown). A second hole 42 which may be of greater diameter is drilled through the tibia 43. A graft or suture 45 which may be of any desired type is anchored by a button or ball 44 on the femur and passed through the holes in the femur and tibia. It is then led through the central passageway in the isometer 1, and secured at the rear of the piston by clamp 27.

The isometer is then adjusted to any desired base tension by appropriate manipulation of the gas regulator 36. Normally this is done with the knee flexed at about 30°. The knee is then moved to various positions and the departure of the piston from the zero position on the scale 9 as noted. If the excursion is substantial, a second small hole may be drilled at a different point in the femur and the exercise repeated until the point of minimum excursion is found.

## Claims

1. An isometric positioner (1) for surgical purposes characterised by a casing (10), a gas pressure chamber (18) in said casing, a piston (19) slidably mounted in said chamber, mean (27) for securing a filament (45) to said piston and gas inlet means (34) for supplying gas under pressure to said chamber (18).

2. A positioner as claimed in claim 1, characterised in that said casing (10) comprises a tapered nose (15, 17) having a passage (22) extending therethrough and connecting with said chamber (18).

3. A positioner as claimed in claim 2, characterised in that the piston (19) comprises a hollow forward extension (20) slidably seated in the passage (22) in said nose (15).

4. A positioner as claimed in any one preceding claim, characterised in that the piston (19) comprises a hollow rear section (24), there being an aperture (25) in the rear of said casing (10) through which said rear section (24) slidingly extends.

5. A positioner as claimed in claim 4, characterised by means (27-32) at the end of said rear section (24) for securing a filament (45) thereto.

6. An isometric positioner (10) for surgical purposes comprising a gas chamber (18) having forward and rear end walls, apertures (22, 25) in said forward and rear walls, a hollow piston (19) slidably positioned in said chamber (18) and having forward (21) and rear (24) tubular extensions slidably passing through said apertures (22, 25), a gas inlet (34) in the forward wall of said chamber (18) and clamp means (32) in the rear extension (24) of said piston (19).

7. A positioner as claimed in any one preceding claim, characterised by means (8) for locking the piston (19) at desired positions in said chamber (18).

8. A positioner as claimed in claim 4, claim 6 or claim 7, characterised by a scale (9) on the rear piston extension (24).

9. A positioner as claimed in any one of claims 6 to 8, characterised by means (35, 36) for supplying gas under pressure to said chamber (18).

10. A positioner as claimed in claim 9, characterised by means (38) for regulating the pressure of the gas supplied to said chamber (18).

0280572

FIG.1

FIG.2

0280572

FIG.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | DE-A-1 958 429  (GAECKEL) <br> * Page 6, line 7 - page 9, line 11; figure 1 * | 1,2,6,8 -10 | A 61 F    2/08 <br> A 61 B   19/00 <br> G 01 L    5/08 <br> G 01 L    7/16 |
| A | --- | 5 | |
| Y | FR-A-2 526 943  (AVL G.F.V.U.M.) <br> * Page 6, lines 22-31; figure * | 1,2,6,8 -10 | |
| A | --- | 3,4 | |
| A,P | EP-A-0 232 049  (PFIZER) <br> * Page 10, lines 27-31; page 11, lines 21-29; figure 7 * | 1,6 | |
| A | GB-A-2 102 351  (JWT) <br> * Page 1, lines 115-126; figures * <br> --- | 8 | |
| A | DE-A-3 411 891  (DAWIDOWSKI) <br> --- | | |
| A | DE-C-  868 045  (POHL) <br> --- | | |
| A | US-A-4 168 621  (KREITENBERG) <br> ----- | | |

TECHNICAL FIELDS
SEARCHED (Int. Cl.4)

A 61 F
A 61 B
G 01 L

The present search report has been drawn up for all claims

| Place of search | Date of completion of the search | Examiner |
|---|---|---|
| THE HAGUE | 02-06-1988 | KLEIN C. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
 
& : member of the same patent family, corresponding
document

EPO FORM 1503 03.82 (P0401)